(19) Europäisches Patentamt
European Patent Office
Office européen des brevets

(11) **EP 3 145 464 B1**

(12) **EUROPÄISCHE PATENTSCHRIFT**

(45) Veröffentlichungstag und Bekanntmachung des Hinweises auf die Patenterteilung:
**22.11.2017 Patentblatt 2017/47**

(21) Anmeldenummer: **15723502.9**

(22) Anmeldetag: **18.05.2015**

(51) Int Cl.:
*A61F 13/534* (2006.01)   *A23B 4/00* (2006.01)
*A61F 13/537* (2006.01)   *A61F 13/15* (2006.01)
*B65D 81/26* (2006.01)

(86) Internationale Anmeldenummer:
**PCT/EP2015/060901**

(87) Internationale Veröffentlichungsnummer:
**WO 2015/177096 (26.11.2015 Gazette 2015/47)**

(54) **VERFAHREN ZUR HERSTELLUNG ZUMINDEST EINER SAUGEINLAGE SOWIE EINE SAUGEINLAGE**

METHOD FOR PRODUCING AT LEAST ONE SUCTION INSERT, AND A SUCTION INSERT

PROCÉDÉ DE PRODUCTION D'AU MOINS UN ÉLÉMENT D'INSERTION ABSORBANT, ET ÉLÉMENT D'INSERTION ABSORBANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.05.2014 DE 102014007209**

(43) Veröffentlichungstag der Anmeldung:
**29.03.2017 Patentblatt 2017/13**

(73) Patentinhaber: **Papierwerk Sundern GmbH**
**59846 Sundern (DE)**

(72) Erfinder: **RAMTHUN, Andreas**
**59755 Arnsberg (DE)**

(74) Vertreter: **Geskes, Christoph**
**Geskes Patent- und Rechtsanwälte**
**Gustav-Heinemann-Ufer 74b**
**50968 Köln (DE)**

(56) Entgegenhaltungen:
**EP-A1- 1 813 239      EP-A1- 2 005 926**
**EP-A2- 0 829 245      CN-U- 201 980 578**
**US-A1- 2008 167 634**

**Beschreibung**

Die Erfindung betrifft ein Verfahren zur Herstellung zumindest einer Saugeinlage sowie eine Saugeinlage.

Saugeinlagen, insbesondere für die Lebensmittelindustrie zur Absorption von überflüssigen Flüssigkeiten in einer Produktverpackung sind aus dem Stand der Technik bekannt. Beispielsweise beschreibt die DE 10 2004 043 289 A1 ein Verfahren zur Herstellung von flüssigkeitsabsorbierenden Saugeinlagen als auch flüssigkeitsabsorbierende Saugeinlagen, wobei zunächst eine erste Materialbahn zu einer Beschickungsstation geführt wird, in der voneinander genau beabstandete Kissen aus Zellstoff und gegebenenfalls Superabsorberpartikeln auf der Materialbahn gebildet werden. Anschließend wird eine zweite Materialbahn parallel zu der ersten Materialbahn zugeführt und miteinander verklebt.

CN 201980578 U beschreibt ein wasserabsorbierendes Kissen, welches ein absorbierendes Material aufweist aus einer Mischung aus Holzfasern und Superabsorber oder absorbierendem Papier, welches Superabsorber aufweist, und welches außenseitig mit Toilettenpapier umhüllt ist.

US 2008/0167634 A1 beschreibt einen absorbierenden Artikel, insbesondere eine Windel, die einen Saugkern aufweist, der mit einem faserigen Material umhüllt ist. Die superabsorbierenden Partikel werden entweder zwischen zwei Lagen der faserigen Materialbahn aufgestreut oder auf eine Materialbahn aufgebracht und mittels einer e-Faltung darin gehalten.

Aufgabe der Erfindung ist es, ein verbessertes Verfahren zur Herstellung einer Saugeinlage zur Verfügung zu stellen. Weiterhin ist Aufgabe der Erfindung eine verbesserte Saugeinlage zur Verfügung zu stellen.

Die Aufgabe wird erfindungsgemäß mittels eines Verfahrens zur Herstellung zumindest einer Saugeinlage nach Anspruch 1 sowie einer Saugeinlage nach Anspruch 15 gelöst. Weitere vorteilhafte Ausgestaltungen sind den nachfolgenden Beschreibungen, den Figuren sowie den Unteransprüchen zu entnehmen. Die einzelnen Merkmale der beschriebenen Ausgestaltungen sind jedoch nicht auf diese beschränkt, sondern können untereinander und mit anderen Merkmalen zu weiteren Ausgestaltungen verknüpft werden.

Es wird ein Verfahren zur Herstellung zumindest einer Saugeinlage vorgeschlagen, das die Schritte umfasst:

- Bereitstellen einer Materialbahn,

- Aufbringen eines Superabsorbers auf die Materialbahn, wobei ein Randbereich senkrecht zur Maschinenrichtung der Materialbahn frei von Superabsorber bleibt,

- Falten der Materialbahn über den Superabsorber, so dass die Materialbahn zumindest teilweise zweilagig ausgestaltet ist, wobei die Materialbahn in einem ersten Schritt v-gefaltet wird und in einem weiteren Schritt nochmals v-gefaltet wird, wobei die Randbereiche nach der ersten Faltung im Wesentlichen übereinanderliegen,

- Falten zumindest eines superabsorberfreien Randbereichs über den zweilagigen Bereich, insbesondere so dass die Materialbahn zumindest vierlagig ausgestaltet ist,

- Kalandrieren, insbesondere satinieren, der gefalteten Materialbahn und

- Kaschieren der gefalteten Materialbahn mit einem Kaschiermaterial.

Im Sinne der Erfindung ist der Randbereich ein Bereich der ungefalteten Materialbahn, der an die Ränder senkrecht zur Maschinenrichtung grenzt. Wird beispielsweise ein Rand senkrecht zur Maschinenrichtung im Wesentlichen auf den gegenüberliegenden Rand gefaltet -V-Faltung - und in einem folgenden Schritt beide Randbereiche zur Faltstelle oder Falz hin zu rück gefaltet, wird ein vierlagiger Saugkern erzeugt. Diese zweimalige V-Faltung wird im Folgenden Doppel-V-Faltung genannt.

Sind im Rahmen der Erfindung Produktionsschritte in zeitlicher Reihenfolge angegeben, so sind damit Produktionsschritte eines Abschnittes des jeweils verwendeten Rohmaterials in Maschinenrichtung zu verstehen.

Im Sinne der Erfindung ist unter einer Materialbahn ein bahnförmiges Flächenprodukt aus Zellstoff, insbesondere Zellstoffwatte oderTissue, aus einem Vlies, aus einem Nonwoven und/oder aus einem gewebten oder gewirkten Material zu verstehen. Das Material umfasst in einer Ausgestaltung zumindest einen Material ausgewählt aus einer Gruppe umfassend einen Zellstoff und/oder ein thermoplastischen Kunststoff, wie beispielsweise ein Polyolefin. Vorteil des vorgeschlagenen Verfahrens ist, dass der auf die Materialbahn aufgebrachte Superabsorber durch das zweifache Falten der Materialbahn in einer Richtung senkrecht zur Maschinenrichtung nicht aus der Saugeinlage herausrieseln kann. Eine nicht erfinderische Faltung ist derart, dass entweder der Randbereich über den Superabsorber gefaltet wird, so dass der zweite Randbereich nicht vom ersten Randbereich überdeckt wird und daraufhin der zweite Randbereich über den ersten Randbereich gefaltet wird. Diese Faltung wird im Folgenden e-Faltung genannt. Eine bevorzugte Möglichkeit der Faltung ist derart, dass der erste Randbereich über den Superabsorber und den zweiten Randbereich gefaltet wird, so dass die beiden Materialbahnkanten im Wesentlichen auf- und/oder übereinander liegen und im Folgenden die beiden übereinanderliegenden Randbereiche noch einmal zurückgeschlagenen beziehungsweise gefaltet werden, so dass die Saugeinlage vierlagig ausgestaltet ist. In einer weiteren Ausgestaltung wird die Falzstelle auf die übereinander liegenden Randbereiche geschlagen. Durch die Faltvorgänge wird vorteilhafterweise erreicht, dass die nun entstandenen Lagen des Saugkerns der Saugeinlage in einem festen Verbund stehen und untereinander nicht verschiebbar sind. In einer weiteren Ausgestaltung wird der Superabsorber derart auf der Materialbahn aufgebracht und insbesondere fixiert, dass dieser im Wesentlichen in der unteren Lage der gefertigten Saugeinlage beziehungsweise des Saugkerns angeordnet ist. Weiterhin vorteilhaft wird der Superabsorber derart auf die Materialbahn aufgebracht und die Materialbahn derart gefaltet, dass der Abschnitt der Materialbahn, auf dem der Superabsorber aufgebracht ist, ausschließlich in Maschinenrichtung bewegt wird. Vorzugsweise wird dieser Abschnitt während des Faltvorgangs nicht hochgefaltet, so dass der Superabsorber im Wesentlichen durch das Falten nicht relativ zur Materialbahn bewegt wird.

Vorzugsweise wird die Materialbahnbreite der ungefalteten Materialbahn auf die Produktbreite des Fertigproduktes eingestellt. Insbesondere beträgt die Gesamtbreite der ungefalteten Materialbahn ein Vielfaches der Saugeinlagenbreite. Beispielsweise wenn die gewünschte Saugeinlagenbreite etwa 9 cm beträgt und der Saugkern soll aus vier Lagen Materialbahn bestehen, so beträgt die Bahnbreite der Zellstoffwattebahn dann etwa das Vierfache, also etwa 36 cm. Die Materialbahn wird hierzu gemäß der Erfindung zweimal gefaltet, das heißt es wird eine Doppel-V-Faltung ausgeführt.

Besonders bevorzugt ist in einer Ausgestaltung vorgesehen, dass beide Randbereiche der Materialbahn frei von Superabsorber bleiben. Dies hat den Vorteil, dass bei der vierlagigen Faltung beziehungsweise der Doppel-V-Faltung, bei der einer der Randbereiche nicht durch die Faltung vom Rest der Materialbahn umschlossen sind, kein Superabsorber an die Umgebung abgeben. Das heißt ein Herausrieselnd des Superabsorbers senkrecht zur Maschinenrichtung wird auch im fertigen Produkt vermieden. Auch wird erreicht, dass bei Verwendung des Superabsorbers insbesondere durch das Quellen des Superabsorbers, der Superabsorber im Saugkern gehalten wird.

Besonders vorteilhaft ist in einer Ausgestaltung vorgesehen, dass der Superabsorber bei Vorsehung der erfinderischen Doppel-V-Faltung nur auf etwa einem Viertel der Bahnbreite der Materialbahn aufgebracht wird. Insbesondere wird der Superabsorber nur auf dem Bereich der Materialbahn aufgebracht, der die untere Lage der gefalteten Materialbahn bildet.

In einer weiteren Ausführungsform ist vorgesehen, dass der Superabsorber durch ein Auftragssystem in einen während des Faltens der Materialbahn geformten Kanal, weiter bevorzugt einen Tunnel geführt oder gefördert wird. Der durch die Materialbahn zumindest teilweise gebildete Kanal ist vorzugsweise einseitig geöffnet. Gebildet wird dieser Kanal beispielsweise durch ein Faltwerk, wobei die Faltung an der Stelle wo der Kanal gebildet ist, beziehungsweise an der der Superabsorber aufgebracht wird, nicht vollständig ausgeführt ist. Vorzugsweise wird direkt nach der Anbringung des Superabsorbers der Faltvorgang beendet und der Superabsorber ist in der gefalteten Materialbahn integriert und insbesondere vorfixiert. In einer bevorzugten Ausgestaltung wird, insbesondere mittels eines Faltwerks, ein Tunnel aus der Materialbahn gebildet. Insbesondere wird eine Faltung teilweise ausgeführt, um den Tunnel zu bilden. In diesen Tunnel wird, vorzugsweise bevor die Faltung vollständig beendet ist, Superabsorber eingebracht. Der Superabsorber wird vorzugsweise in Maschinenrichtung von Hinten in den Tunnel eingebracht. Weiterhin ist in einer Ausgestaltung vorgesehen, dass das Auftragssystem ausgestaltet ist als Lanze, Schwert, Luftstrom oder Rollband.

In einer weiteren bevorzugten Ausgestaltung ist vorgesehen, dass der Superabsorber derart auf der Materialbahn fixiert ist, dass er vorzugsweise auch nach der Faltung auf und/oder in der unteren Lage des gefertigten Saugkerns der Saugeinlage angeordnet ist. Hierdurch wird vorteilhafterweise erreicht, dass die aufzunehmende Flüssigkeit sofort den Superabsorber erreicht und dieser die Flüssigkeit umgehend aufnehmen kann. Weiterhin bevorzugt werden die übrigen Lagen der Materialbahn über dem Superabsorber angeordnet und sind derart ausgelegt, dass diese durch die Kapillarwirkung für ein gleichmäßiges Verteilen der Flüssigkeit im Saugkern wirken, insbesondere um die volle Wirkung des Superabsorbers ausschöpfen zu können.

Werden im Rahmen der Erfindung die Begriffe "unten" und "oben" verwendet, so geben diese lediglich relative Bezüge zueinander an. Vorzugsweise verweisen Richtungsbezeichnungen auf die Richtungen im Produktionsprozess, bevorzugt bezogen auf die Maschinenrichtung.

Im Rahmen der Erfindung ist unter einem Saugkern die gefaltete Materialbahn zu verstehen, die insbesondere auch den Superabsorber einschließt. Die Saugeinlage hingegen umfasst den Saugkern und darüber hinaus noch weitere Komponenten, wie beispielsweise eine Kaschierung.

In einer weiteren Ausführungsform ist vorgesehen, dass der Superabsorber beim Kalandrieren des Saugkerns zerkleinert, insbesondere zermahlen, wird. Das Kalandrieren beziehungsweise Satinieren des Saugkerns hat den Vorteil, dass eine kompakte Einheit von Materialbahn und Superabsorber erzielt wird, wobei sich der zerkleinerte Superabsorber in der Materialbahn verankert. Weiterhin vorteilhaft an der Zerkleinerung des Superabsorbers ist, dass dieser eine größere Oberfläche zur Aufnahme von Flüssigkeit aufweist, als der unzerkleinerte Superabsorber. Weiterhin vorteilhaft ist, dass der Superabsorber relativ grob auf die Materialbahn aufgebracht werden kann, was eine Handhabung und insbesondere eine höhere Maschinengeschwindigkeit erlaubt; dennoch sind die Vorteile eines relativ feinkörnigen Superabsorbers im Saugkern beziehungsweise in der Saugeinlage nutzbar. Weiter vorteilhaft ist in einer Ausgestaltung vorgesehen, dass der Superabsorber mittels der Kalandrierung in der Materialbahn verankert wird. Bevorzugt erfolgt eine mechanische Verhaftung zwischen dem Superabsorber und dem Material der Bahn.

In einer weiteren Ausgestaltung wird vorgeschlagen, dass die Materialbahn angefeuchtet wird, insbesondere bevor der Superabsorber auf die Materialbahn aufgebracht wird. Dies hat den Vorteil, dass der Superabsorber aktiviert wird und sich in den Fasern der Materialbahn verankert. In einer weiteren Ausgestaltung ist vorgesehen, dass die Materialbahn im Produktionsprozess trocken bleibt, das heißt einen Feuchtegehalt nach DIN 54540 Teil 4, Stand Juni 1989, von etwa 0 % bis etwa 10 %, bevorzugt etwa 3 % bis etwa 8 %, aufweist.

In einer weiteren Ausgestaltung ist vorgesehen, dass das Kaschiermaterial zumindest ein Material ausgewählt aus einer Gruppe umfassend eine Folie, ein Gewebe und/oder ein Gewirke ist. Insbesondere umfasst das Kaschiermaterial Polyethylen, Polypropylen und/oder Papier. Weiterhin vorteilhafterweise wird gemäß einer Ausführungsform das Kaschiermaterial aus einem Gewebe oder Gewirke hergestellt. Weiterhin bevorzugt ist das Kaschiermaterial derart gestaltet, dass dieses hydrophil wirkt. Beispielsweise umfasst das Kaschiermaterial eine Folie mit Ventilwirkung und/oder ein hydrophil ausgestaltetes Nonwoven-Material, damit die aufzunehmende Flüssigkeit den Saugkern möglichst schnell erreicht und durch den eingelagerten Superabsorber gebunden wird. Eine Ventilwirkung des Kaschiermaterials wird insbesondere durch Öffnungen erzielt, die beispielsweise mittels einer Nadelung erzeugt werden, die jeweils eine Kapillarwirkung aufweisen.

In einer Ausführungsform ist vorgesehen, dass das Kaschiermaterial beispielsweise über Formschulterbleche als Schlauch geformt und um den Saugkern gelegt wird. Vorteilhafterweise wird das Kaschiermaterial zumindest teilweise mit einem Kleber beispielsweise einem Hotmelt benetzt. Weiterhin vorteilhaft wird durch ein anschließendes Faltwerk beispielweise in Verbindung mit einem Prägewerk ein Verbund des Kaschiermaterials mit dem Saugkern gebildet. Durch diesen Fertigungsvorgang ist der Saugkern vorteilhafterweise ringsum verschlossen, so dass gänzlich verhindert wird, dass Superabsorber aus der Saugeinlage heraustritt.

In einer weiteren Ausführungsform ist vorgesehen, dass das Kaschiermaterial über insbesondere Formschulterbleche als Halbschlauch gebildet wird und teilweise um den Saugkern gelegt wird. Vorzugsweise wird in einem weiteren Schritt oder gleichzeitig eine weitere Bahn umfassend eine Folie, ein Nonwoven und/oder ein nassfestes Papier als Abdecklage aufkaschiert.

In einer Ausführungsform des Verfahrens ist vorgesehen, dass das Kaschiermaterial eine Anzahl von Saugeinlagen verbindet. Unter anderem ist vorgesehen, dass das Kaschiermaterial Perforierungen zwischen den Saugeinlagen aufweist. Gemäß einer Ausgestaltung ist vorgesehen, dass die Saugkerne vor der Kaschierung von einander getrennt werden, beziehungsweise die gefaltete und gegebenenfalls kalandrierte Materialbahn geschnitten wird und die einzelnen Saugkerne von einander beabstandet sind, dass diese vorzugsweise von dem Kaschiermaterial umschlossen werden und in dem nachfolgenden Schritten die Saugeinlagen vereinzelbar sind.

Die miteinander insbesondere über das Kaschiermaterial verbundenen Saugeinlagen werden gemäß einer weiteren Ausführungsform aufgerollt. In einer weiteren Ausgestaltung ist vorgesehen, dass die einzelnen Saugeinlagen von einander getrennt werden, in dem das Kaschiermaterial durchtrennt wird.

Eine weitere Ausführungsform sieht vor, dass ein Saugkern umfassend die Materialbahn und den Superabsorber, zumindest in Maschinenrichtung kürzer ist als die Kaschierung.

Weiterhin wird eine Saugeinlage mit einem Saugkern und einem Kaschiermaterial vorgeschlagen, wobei der Saugkern eine gefaltete Materialbahn und einen Superabsorber aufweist.

Die Materialbahn ist um den auf der Materialbahn aufliegenden Superabsorber herum gefaltet, so dass der Superabsorber von zumindest einer Seite zumindest dreilagig, vorzugsweise vierlagig von der Materialbahn bedeckt ist, wobei die Materialbahn doppelt v-gefaltet ist, wobei der Saugkern kalandriert ist und das Kaschiermaterial um den Saugkern gelegt ist.

Vorteilhafterweise wird die Saugeinlage für Lebensmittel insbesondere für Lebensmittelverpackung oder für industrielle Anwendungen, bei der eine Flüssigkeitsaufnahme vorgesehen ist, verwendet. Gemäß einer Ausgestaltung ist über die Aufbringung von Superabsorbern und der Auswahl einer Zellstoffwatte und/oder eines Vlies für die Materialbahn eine Flüssigkeitsaufnahme eingestellt. Weiterhin vorteilhaft ist das Produktgewicht über die Masse der Materialbahn beziehungsweise Zellstoffwatte und die Menge des aufgebrachten Superabsorbers sowie des eingesetzten Kaschiermaterials eingestellt. Weiterhin ist gemäß einer Ausgestaltung vorgesehen, dass das Volumen des Saugkerns der Saugeinlage über den Kreppungsgrad der Zellstoffwatte der Materialbahn, der Anzahl der Faltungen der Materialbahn, der Menge des Superabsorber und/oder der nachgeschalteten Satinage eingestellt ist.

In einer Ausgestaltung ist vorgesehen, dass der Superabsorber zumindest ein querverbundenes Natriumpolycarbonat umfasst. Weiterhin ist in einer weiteren Ausgestaltung vorgesehen, dass der Superabsorber eine Zentrifugenspeicherfähigkeit (centrifuge retention capacity) nach WSP 241.3.R (12) von etwa 20 g/g bis etwa 45 g/g, bevorzugt etwa 30 g/g bis etwa 40 g/g, weiter bevorzugt etwa 32 g/g bis etwa 34 g/g, weiter bevorzugt etwa 33,5 g/g aufweist. Weiterhin ist gemäß einer Variante vorgesehen, dass der Superabsorber eine Absorptionsrate unter einem Druck von 4,83 kPa (0,7 psi) nach WSP 242.3.R (12) von etwa 15 g/g bis etwa 35 g/g, bevorzugt etwa 20 g/g bis etwa 25 g/g, weiter bevorzugt etwa 22 g/g aufweist. Weiterhin ist gemäß einer Ausgestaltung vorgesehen, dass der Superabsorber eine Partikelgrößenverteilung nach WSP 220.3.R3 (12) von größer 300 $\mu$m entspricht etwa 40 % bis etwa 60 %, bevorzugt etwa 45 % bis etwa 55 %, weiter bevorzugt etwa 50 %, vorzugsweise größer 850 $\mu$m entspricht etwa 0,1 % bis etwa 1,5 %, bevorzugt 0 % bis etwa 0,5 %, weiter bevorzugt maximal 1 % aufweist. In einer weiteren bevorzugten Ausgestaltung ist vorgesehen, dass der Superabsorber ein Superabsorber der Firma Evonik Industries AG in 47508 Krefeld, Deutschland, mit dem Markennamen "FAVOR SXM 9155" ist. Weiterhin vorteilhaft ist der Superabsorber ein Superabsorber mit etwa den Kenndaten des "FAVOR SXM 9155" von November 2013.

Die Materialbahn umfasst vorzugsweise eine Hochkreppprägewatte, eine Prägewatte und/oder eine Formatware. Vorzugsweise ist die Materialbahn hochgebleicht.

Ist im Rahmen der Erfindung der Begriff "etwa" verwendet, insbesondere in Bezug auf Werte und/oder Wertebereiche, so ist darunter ein Toleranzbereich zu verstehen, den der Fachmann auf diesem Gebiet für üblich erachtet, insbesondere ist ein Toleranzbereich von +/- 20 %, bevorzugt +/- 10 %, noch weiter bevorzugt +/- 5 %, vorgesehen. Ist im Rahmen der Erfindung der Begriff "im Wesentlichen" verwendet, so ist damit eine Toleranz zu verstehen, unter der der Fachmann unter technischen und wirtschaftlichen Gesichtspunkten das angegebene Merkmal als solches erkennt.

In einer weiteren Ausgestaltung ist vorgesehen, dass die Flächenmasse der Materialbahn bevor diese zum Saugkern verarbeitet wird nach DIN EN 12625 von 2011 zwischen etwa 18 g/m$^2$ und etwa 30 g/m$^2$, bevorzugt etwa 20 g/m$^2$ bis etwa 25 g/m$^2$ umfasst. In einer weiteren Ausgestaltung ist vorgesehen, dass die Flächenmasse nach DIN EN126256 von 2011 zwischen etwa 180 g/m$^2$ und etwa 260 g/m$^2$, bevorzugt etwa 200 g/m$^2$ bis etwa 250 g/m$^2$, weiter bevorzugt etwa 220 g/m$^2$ bis etwa 260 g/m$^2$ beträgt. Vorzugsweise beträgt die Flächenmasse etwa 210 g/m$^2$ bis etwa 230 g/m$^2$. Der Bereich zwischen etwa 18 g/m$^2$ und etwa 30 g/m$^2$ der Flächenmasse für die Materialbahn bezieht sich insbesondere auf einlagiges Hochkrepp oder Prägewatte oder Formatware. Der Bereich zwischen etwa 180 g/m$^2$ und etwa 260 g/m$^2$ bezieht sich insbesondere auf Prägewatte, Hochkrepp oder Formatware, die mehrlagig einem Kalender vor der Weiterverarbeitung zum Saugkern zugeführt wurde. Vorzugsweise weist die Materialbahn vor der Weiterverarbeitung eine Wasseraufnahme bei 500 g Belastung von mindestens 17 g, vorzugsweise etwa 17 g bis etwa 20 g, weiter bevorzugt etwa 17 g bis etwa 50 g auf. Weiterhin bevorzugt weist die Materialbahn vor Weiterverarbeitung eine Wasseraufnahme bei 1000 g Belastung von mindestens 14 g, bevorzugt etwa 14 g bis etwa 20 g, weiter bevorzugt etwa 14 g bis etwa 50 g auf.

Vorzugsweise weist die Materialbahn eine freie Wasseraufnahme von zumindest 21 g, bevorzugt etwa 21 g bis etwa 30 g, weiter bevorzugt etwa 21 g bis etwa 50 g auf.

Zur Bestimmung der Wasseraufnahme der Materialbahn wird eine Probe der Materialbahn von 80 mm * 120 mm, die vorher auf 0,01 g genau eingewogen wurde - was die klimatisierte Masse $M_k$ ist, vollflächig mit der Saugseite nach unten in eine mit entionisiertem Wasser gefüllte Schale gelegt und vollständig untergetaucht. Die Bewässerungsdauer beträgt 60 Sekunden. Die Probe befindet sich während des Wässerns unterhalb des Wasserspiegels, wobei ein allseitiger Wasserzutritt zur Probe sichergestellt ist. Nach der Bewässerungszeit von 60 Sekunden wird die Probe mit der Schmalseite parallel zu einer Tischoberfläche mit einer Breitklemme für 30 Sekunden zum Abtropfen aufgehangen. Hierbei wird ein Übereinanderschlagen der Probe vermieden. Nach einer Abtropfzeit von 30 Sekunden wird die Probe auf 0,01 g genau ausgewogen - was der Masse nass $M_n$ entspricht. Die Die freie Wasseraufnahme WA berechnet sich dann wie folgt:

$$WA = M_n - M_k$$

Zur Bestimmung der Wasseraufnahme $WA_{bel}$ unter Belastung wird nach einer Bewässerungsdauer von 30 Sekunden und einer Ablaufzeit von 15 Sekunden eine Probe mit den Maßen 80 mm * 120 mm bestimmt. Die Probe wird in einer Polystyrolschale mit der Saugseite nach unten eingelegt. Schale und Proben werden gemeinsam zur Bestimmung der Masse $M_k$ auf 0,01 g genau ausgewogen. Anschließend wird die Probe mit 20 g entionisiertem Wasser umspült. Nachdem eine Flüssigkeitsmenge von der Probe aufgenommen wurde, wird in das Gewicht von 500 g beziehungsweise 1000 g vollflächig aufgebracht. Die Probe verbleibt 30 Sekunden in horizontaler Stellung. Im Folgenden wird die Schale in Schrägstellung gebracht, das heißt mit einer Ablaufvorrichtung mit 25 °Neigungswinkel aufgesetzt. Nach 50 Sekunden wird die Flüssigkeitsmenge die sich im Schalenboden gesammelt hat mit einem Saugtuch abgesaugt, die Probe wird entlastet und zur Bestimmung der Masse $M_n$ auf 0,01 g gewogen. Die Wasseraufnahme unter Belastung $WA_{bel}$ berechnet sich dann wie folgt:

$$WA_{bel} = M_n - M_k$$

In einer weiteren Ausgestaltung ist vorgesehen, dass Superabsorber von zumindest einer Seite zumindest dreilagig von der Materialbahn bedeckt ist.

Eine weitere Variante sieht vor, dass die Materialbahnen doppelt-V-gefaltet ist. Wie bereits weiter oben erwähnt, hat diese doppelte V-Faltung, das heißt die Materialbahn wird vorgefaltet und in einem nachfolgenden Schritt wird die vorgefaltete Materialbahn nochmals V-gefaltet, den Vorteil, dass der Superabsorber sicher in den Saugkern eingeschlossen ist.

Zur weiteren Vermeidung des Heraustretens des Superabsorbers aus der Saugeinlage und zur Vermeidung einer Kontamination von Lebensmitteln mit Superabsorber ist der Saugkern mit einem Kaschiermaterial kaschiert. Vorzugsweise ist das Kaschiermaterial zumindest ein Material ausgewählt aus einer Gruppe umfassend eine Folie, ein Gewebe und/oder ein Gewirke. Beispielsweise umfasst das Kaschiermaterial Polyethylen, Polypropylen und/oder Papier.

Gemäß einer weiteren Ausführungsform ist vorgesehen, dass die Saugeinlage mit weiteren Saugeinlagen mittels der Kaschierung verbunden ist.

Dies wird insbesondere dadurch erreicht, dass die Kaschierung ein fortlaufendes Material ist, das bei der Produktion um die vereinzelten Saugkerne gelegt wird und/vorzugsweise geprägt oder derart verschweißt wird, dass die einzelnen Saugeinlagen nicht von einander oder nicht vollständig von einan der getrennt sind.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zur weiteren Ausgestaltungen kombiniert werden.

Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:

Fig. 1 die Faltung eines Saugkerns in Doppel-V-Faltung;

Fig. 2 die Faltung eines nicht erfinderischen Saugkerns in e-Faltung;

Fig. 3 ein schematischen Querschnitt durch eine erste Saugeinlage;

Fig. 4 einen schematischen Querschnitt durch eine zweite Saugeinlage;

Fig. 5 eine Aufsicht auf eine Saugeinlage; und

Fig. 6 eine schematische Darstellung des Produktionsprozesses zur Herstellung einer Saugeinlage.

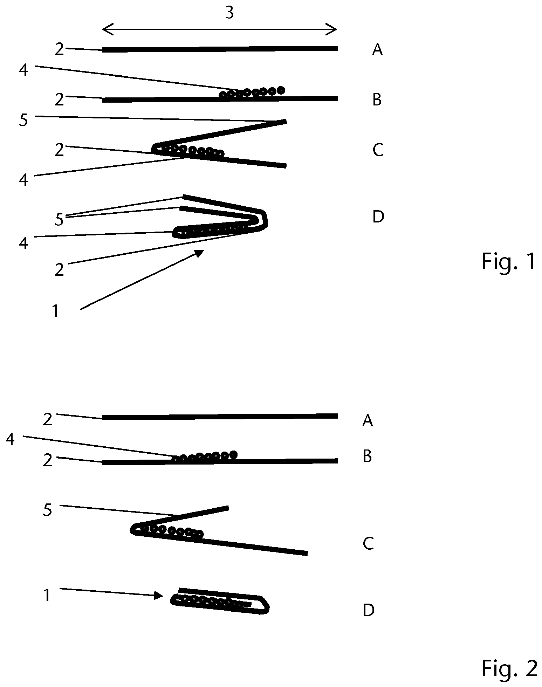

Fig. 1 zeigt die schematische Darstellung einer Faltung eines Saugkerns 1 umfassend eine Materialbahn 2 mit einer definierten Breite 3, die ausgewählt ist, dass durch den Faltvorgang die gewünschte Breite des Saugkerns 1 erzielt wird. Beispielsweise weist die Materialbahn 2 eine Breite 3 senkrecht zur Maschinenrichtung von etwa 36 cm auf. Im Schritt B wird auf die Materialbahn 2 ein Superabsorber 4 vorzugsweise in Granulat- oder Pulverform auf die Materialbahn 2 aufgebracht. Vorzugsweise wird der Superabsorber 4 nur auf einem Viertel der Breite der Materialbahn 2 aufgebracht. Vorzugsweise kann die Aufbringung des Superabsorbers 4 auf die Materialbahn 2 gleichzeitig mit der Faltung der Materialbahn einhergehen, die beispielsweise im Schritt C zu erkennen ist. Das Einbringen des Superabsorbers 4 geschieht gemäß einer Ausgestaltung mittels eine Lanze oder eines Schwertes, die in die halbgefaltete Materialbahn 2, wie sie in Schritt C dargestellt ist, eingeführt wird, so dass die Materialbahn 2 durch ein hier nicht dargestelltes Faltwerk in Maschinenrichtung hinter der Einbringlanze für den Superabsorber 4 fertiggefaltet wird. In Schritt D ist zu erkennen, dass nach der erfolgten V-Faltung in Schritt C die Materialbahn 2 nochmals V-gefaltet wird, um so eine Doppel-V-Faltung zu erzielen. Der so gestaltete Saugkern 1 wird in folgenden Schritten weiterverarbeitet.

Fig. 2 zeigt eine nicht erfinderische Faltung der Materialbahn zur Herstellung eines Saugkernes. Die Materialbahn 2 aus Schritt A wird im folgenden Schritt B mit dem Superabsorber versehen und danach oder gleichzeitig ein Randbereich 5 über den Superabsorber 4 gefaltet. Der Schritt D zeigt den in e-Form fertig gefalteten Saugkern 1, der in folgenden Schritten zu einer Saugeinlage weiter verarbeitet wird.

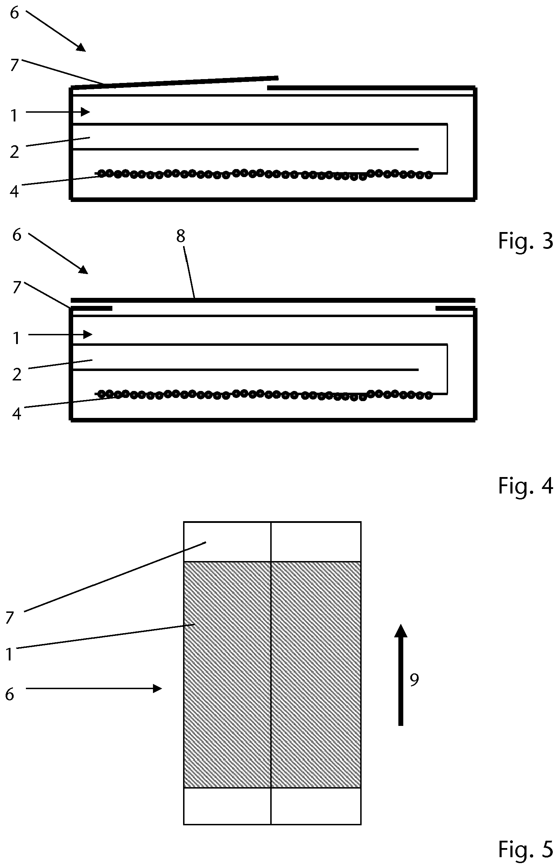

Fig. 3 zeigt einen schematischen Querschnitt durch eine Saugeinlage 6 dessen Materialbahn 2 eine Doppel-V-Faltung aufweist. Zu erkennen ist, dass der Superabsorber 4 auf der hier auf der untersten Lage der Materialbahn 2 beziehungsweise des Saugkernes 1 aufliegt beziehungsweise zwischen einer ersten und einer zweiten Lage angeordnet ist und zumindest teilweise in die Materialbahn 2 eingedrungen ist. Die Saugeinlage 6 umfasst des Weiteren ein Kaschiermaterial 7, das wie in Fig. 3 schematisch gezeigt, um den Saugkern 1 herum gefaltet ist. Das Kaschiermaterial 7 ist vorzugsweise flüssigkeitsdurchlässig und insbesondere genadelt. Besonders bevorzugt ist vorgesehen, dass das Kaschiermaterial 7 eine hydrophobe, genadelte Folie umfasst. Zur Kaschierung des Saugkerns 1 wird das Kaschiermaterial 7 über Formschulterbleche als Schlauch beziehungsweise schlauchähnlich gebildet und um den Saugkern 1 gelegt. Das Kaschiermaterial 7 wird vollständig oder teilweise mit Kleber benetzt, so dass durch das anschließende Folienfaltwerk gegebenenfalls in Verbindung mit einem Prägewerk ein Verbund mit dem Saugkern 1 gebildet wird. Ein solcher Fertigungsvorgang bewirkt, dass der Saugkern 1 ringsherum von dem Kaschiermaterial 7 umschlossen ist.

Fig. 4 zeigt eine weitere Variante der Kaschierung des Saugkerns 1 zur Herstellung einer Saugeinlage 6, bei dem das Kaschiermaterial 7 beispielsweise über Formschulterbleche zu einem halbschlauchähnlichen Gebilde geformt wird und um den Saugkern 1 gelegt werden. Eine Abdecklage 8 die eine Folie, ein Nonwoven-Material oder ein nassfestes Papier beziehungsweise Tissue aufweist, wird gleichzeitig oder in einem folgenden Schritt zumindest auf die kaschierungsfreie Fläche aufkaschiert. Dies kann beispielsweise durch ein Aufbringen eines Klebers oder durch Aufschmelzen der Abdecklage 8 und/oder des Kaschiermaterials 7 beziehungsweise mittels Ultraschallverschweißung oder Prägen erfolgen.

Fig. 5 zeigt in einer Aufsicht eine Saugeinlage 6. Die Saugeinlage 6 weist ein Saugkern 1 auf, der in Maschinenrichtung 9 kürzer ist, als die Kaschierung 7.

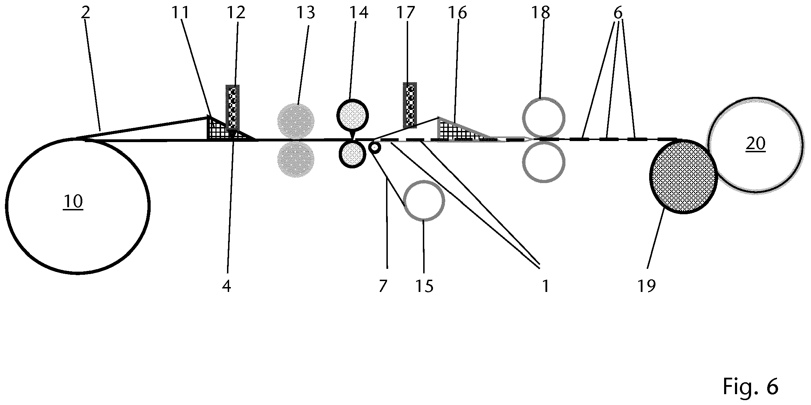

Fig. 6 zeigt in schematischer Darstellung den Produktionsprozess zur Herstellung von Saugeinlagen 6. Eine Materialbahn 2 wird von einer Ursprungsrolle 10 abgewickelt und mittels eines Materialbahnfaltwerks 11 gefaltet. Bevor oder während der Faltung wird ein Superabsorber 4 in Granulat- oder Pulverform auf die Materialbahn 2 aufgebracht und durch das Falten der Materialbahn 2 auf der Materialbahn 2 fixiert. Die Einbringung des Superabsorbers 4 geschieht mittels eines Superabsorberapplikators 12. In den nachfolgenden Schritt wird die fertiggefaltete Materialbahn 2 durch Satinage-Walzen geführt, wobei die Materialbahn 2 satiniert und vorzugsweise der Superabsorber 4 zermalmt wird. Der so entstandene Saugkern 1 wird mittels einer Schneidevorrichtung 14 vereinzelt. Im Folgendem wird Kaschiermaterial 7 von einer Folienrolle 15 in den Produktionsprozess eingebracht und durch ein Folienfaltwerk 16 derart gefaltet, dass sich das Kaschiermaterial 7 um die vereinzelten Saugkerne 1 legt. Zur späteren Verklebung des Kaschiermaterials 7 wird ein Klebstoff beispielsweise ein Hotmelt mittels eines Klebstoffapplikators 17 auf das Kaschiermaterial 7 aufgebracht. Nach dem Falten des Kaschiermaterials 7 um die vereinzelten Saugkerne 1 wird das Produkt durch ein Prägewerk 18 geführt, dass die Saugeinlage 6 fertigstellt. Das Prägewerk 18 kann insbesondere eine Perforierung oder einer Prägung weiterhin bevorzugt einer Verschweißung in Maschinenrichtung vor und hinter dem einzelnen Saugkernen 1 des Kaschiermaterials 7 bewirken. Vorzugsweise wird das Kaschiermaterial 7 von der Folienrolle 15 bereits perforiert in den Prozess eingebracht. Im Folgenden werden die fertigen Saugeinlagen 6, die noch miteinander verbunden sind, mittels einer Aufrollung 19 auf eine Fertigrolle 20 aufgerollt. Die Saugeinlage 6 kann in einer hier nicht dargestellten Ausgestaltung auch nach dem Prägewerk 18 vereinzelt werden beispielsweise durch eine Schneidevorrichtung. Somit können die fertigen Saugeinlagen 6 als einzelne Formate oder als Rollenware gefertigt werden.

**Patentansprüche**

1. Verfahren zur Herstellung zumindest einer Saugeinlage (6) umfassend die Schritte Bereitstellen einer Materialbahn (2),
Aufbringen eines Superabsorbers (4) auf die Materialbahn (2), wobei zumindest ein Randbereich (5) senkrecht zur Maschinenrichtung der Materialbahn (2) frei von Superabsorber (4) bleibt,
Falten der Materialbahn (2) über den Superabsorber (4), so dass die Materialbahn (2) zumindest teilweise zweilagig ausgestaltet ist,
Falten zumindest des zumindest einen superabsorber-freien Randbereiches (5) über den zweilagigen Bereich, wobei die Materialbahn (2) in einem ersten Schritt v-gefaltet wird und in einem weiteren Schritt nochmals v-gefaltet wird, wobei die Randbereiche (5) nach der ersten Faltung im Wesentlichen übereinanderliegen,
Kalandrieren der gefalteten Materialbahn (2),
Kaschieren der gefalteten Materialbahn (2) mit einem Kaschiermaterial (7).

2. Verfahren nach Anspruch 1, **dadurch gekennzeichnet, dass** beide Randbereiche (5) der Materialbahn (2) frei von Superabsorber (4) bleiben.

3. Verfahren nach einem der vorhergehenden Ansprüche, **dadurch gekennzeichnet, dass** der Superabsorber (4) beim Kalandrieren zerkleinert wird.

4. Verfahren nach einem der vorhergehenden Ansprüche, **dadurch gekennzeichnet, dass** die Materialbahn (2) angefeuchtet wird.

5. Verfahren nach einem der vorhergehenden Ansprüche, **dadurch gekennzeichnet, dass** das Kaschiermaterial (7) zumindest ein Material ausgewählt aus einer Gruppe umfassend eine Folie, ein Gewebe, ein Vlies und/oder ein Gewirke ist.

6. Verfahren nach Anspruch 5, **dadurch gekennzeichnet, dass** das Kaschiermaterial (7) ein hydrophobes Material und/oder ein Material mit Ventilwirkung umfasst.

7. Verfahren nach einem der vorhergehenden Ansprüche, **dadurch gekennzeichnet, dass** das Kaschiermaterial (7) eine Anzahl von Saugeinlagen (6) verbindet.

8. Verfahren nach Anspruch 7, **dadurch gekennzeichnet, dass** eine Anzahl von Saugeinlagen (6) aufgerollt werden.

9. Verfahren nach einem der vorhergehenden Ansprüche, **dadurch gekennzeichnet, dass** die einzelnen Saugeinlagen (6) voneinander getrennt werden, indem das Kaschiermaterial (7) durchtrennt wird.

10. Verfahren nach einem der vorhergehenden Ansprüche, **dadurch gekennzeichnet, dass** ein Saugkern (1), umfassend die Materialbahn (2) und den Superabsorber (4), in Maschinenrichtung kürzer ist als die Kaschierung (7).

11. Saugeinlage (6), hergestellt nach einem Verfahren nach einem oder mehreren der Ansprüche 1 bis 10, mit einen Saugkern (1) und einem Kaschiermaterial (7), wobei der Saugkern (19) eine gefaltete Materialbahn (2) und einen Superabsorber (4) aufweist, wobei die Materialbahn (2) um den auf der Materialbahn (2) aufliegenden Superabsorber (4) herumgefaltet ist, so dass der Superabsorber (4) von zumindest einer Seite zumindest dreilagig von der Materialbahn (2) bedeckt ist, wobei die Materialbahn (2) doppelt v-gefaltet ist, wobei der Saugkern (1) kalandriert ist und das Kaschiermaterial (7) um den Saugkern (1) gelegt ist.

12. Saugeinlage (6) nach Anspruch 11, **dadurch gekennzeichnet, dass** die Saugeinlage (6) nur eine Schicht Superabsorber (4) aufweist.

13. Saugeinlage (6) nach einem der Ansprüche 11 oder 12, **dadurch gekennzeichnet, dass** das Kaschiermaterial (7) zumindest ein Material umfasst ausgewählt aus einer Gruppe umfassend eine Folie, ein Gewebe, ein Vlies und/oder ein Gewirke.

14. Saugeinlage (6) nach einem der Ansprüche 11 bis 13, **dadurch gekennzeichnet, dass** die Saugeinlage (6) mit weiteren Saugeinlagen (6) mittels der Kaschierung (7) verbunden ist.

**Claims**

1. Method for manufacturing at least one absorbent pad (6) comprising the steps of providing a material web (2),
applying a super absorbent (4) onto the material web (2), wherein at least one margin area (5) perpendicular to the machine direction of the material web (2) remains free of the super absorbent (4),
folding of the material web (2) via the superabsorbent (4), in a way that the material web (2) is at least partially formed in two layers,
folding at least of the at least one superabsorbent-free margin area (5) over the two-layered area, wherein the material web (2) is folded in v-form in the first step and folded in v-form again in a further step, wherein the margin areas (5) are substantially lying on top of each other after the first folding,
calendering of the folded material web (2),
laminating of the folded material web (2) with a laminating material (7).

2. Method according to claim 1, **characterized in that** the two margin areas (5) of the material web (2) remain free of superabsorbent (4).

3. Method according to one of the preceding claims, **characterized in that** the superabsorbent (4) is crushed during calendering.

4. Method according to one of the preceding claims, **characterized in that** the material web (2) is moistened.

5. Method according to one of the preceding claims, **characterized in that** the laminating material (7) is at least a material selected from a group comprising a film, a tissue, a fleece and/or a knitting.

6. Method according to claim 5, **characterized in that** the laminating material (7) comprises a hydrophobe material and/or a material having a valve effect.

7. Method according to one of the preceding claims, **characterized in that** the laminating material (7) connects a number of absorbent pads (6).

8. Method according to claim 7, **characterized in that** a number of absorbent pads (6) is rolled up.

9. Method according to one of the preceding claims, **characterized in that** the single absorbent pads (6) are separated of each other by cutting through the laminating material (7).

10. Method according to one of the preceding claims, **characterized in that** the absorbent core (1), comprising the material web (2) and the super absorbent (4), is shorter then the lamination (7) in machine direction.

11. Absorbent pad (6), manufactured according to a method according to one or more of the claims 1 to 10, with an absorbent core (1) and a laminating material (7), wherein the absorbent core (1) has a folded material web (2) and a super absorbent (4), wherein the material web (2) is folded around the superabsorbent (4) resting on the material web (2) in a way that the superabsorbent (4) is on at least one side covered by at least three layers of the material web (2), wherein the material web (2) is double fold in v-form, wherein the absorbent core (1) is calendered and the laminating material (7) is applied around the absorbent core (1).

12. Absorbent pad (6) according to claim 11, **characterized in that** the absorbent pad (6) comprises only one layer of the super absorbent (4).

13. Absorbent pad (6) according to one of the claims 11 or 12, **characterized in that** the laminating material (7) comprises at least one material selected from a group comprising a film, a tissue, a fleece and/or a knitting.

**14.** Absorbent pad (6) according to one of the claims 11 to 13, **characterized in that** the absorbent pad (6) is connected with further absorbent pads (6) by means of the lamination (7).

**Revendications**

**1.** Procédé de production d'au moins un élément d'insertion absorbant (6) comprenant les étapes suivantes:

préparer une bande de matière (2),
déposer un super-absorbeur (4) sur la bande de matière (2), dans lequel au moins une région de bord (5) perpendiculairement à la direction machine de la bande de matière (2) reste libre de super-absorbeur (4),
plier la bande de matière (2) sur le super-absorbeur (4), de telle manière que la bande de matière (2) soit configurée au moins partiellement à deux couches,
plier au moins ladite au moins une région de bord (5) libre de super-absorbeur sur la région à deux couches, dans lequel la bande de matière (2) est pliée en v dans une première étape et est encore pliée en v dans une autre étape, dans lequel les régions de bord (5) sont essentiellement superposées après le premier pliage,
calandrer la bande de matière pliée (2),
contrecoller la bande de matière pliée (2) avec une matière de doublure (7).

**2.** Procédé selon la revendication 1, **caractérisé en ce que** les deux régions de bord (5) de la bande de matière (2) restent libres de super-absorbeur (4).

**3.** Procédé selon l'une quelconque des revendications précédentes, **caractérisé en ce que** l'on broie le super-absorbeur (4) lors du calandrage.

**4.** Procédé selon l'une quelconque des revendications précédentes, **caractérisé en ce que** l'on humidifie la bande de matière (2).

**5.** Procédé selon l'une quelconque des revendications précédentes, **caractérisé en ce que** la matière de doublure (7) est au moins une matière choisie dans un groupe comprenant une feuille, un tissu, un non-tissé et/ou un tricot.

**6.** Procédé selon la revendication 5, **caractérisé en ce que** la matière de doublure (7) comprend une matière hydrophobe et/ou une matière avec une action de soupape.

**7.** Procédé selon l'une quelconque des revendications précédentes, **caractérisé en ce que** la matière de doublure (7) relie un certain nombre d'éléments d'insertion absorbants (6).

**8.** Procédé selon la revendication 7, **caractérisé en ce qu'**un certain nombre d'éléments d'insertion absorbants (6) sont enroulés.

**9.** Procédé selon l'une quelconque des revendications précédentes, **caractérisé en ce que** les éléments d'insertion absorbants individuels (6) sont séparés l'un de l'autre, du fait que la matière de doublure (7) est coupée.

**10.** Procédé selon l'une quelconque des revendications précédentes, **caractérisé en ce qu'**un noyau absorbant (1), comprenant la bande de matière (2) et le super-absorbeur (4), est plus court dans la direction machine que la doublure (7).

**11.** Elément d'insertion absorbant (6), produit par un procédé selon une ou plusieurs des revendications 1 à 10, avec un noyau absorbant (1) et une matière de doublure (7), dans lequel le noyau absorbant (19) présente une bande de matière pliée (2) et un super-absorbeur (4), dans lequel la bande de matière (2) est repliée autour du super-absorbeur (4) déposé sur la bande de matière (2), de telle manière que le super-absorbeur (4) soit couvert par au moins un côté au moins à trois couches de la bande de matière (2), dans lequel la bande de matière (2) est doublement pliée en v, dans lequel le noyau absorbant (1) est calandré et la matière de doublure (7) est posée autour du noyau absorbant (1).

**12.** Elément d'insertion absorbant (6) selon la revendication 11, **caractérisé en ce que** l'élément d'insertion absorbant (6) ne présente qu'une couche de super-absorbeur (4).

**13.** Elément d'insertion absorbant (6) selon une des revendications 11 ou 12, **caractérisé en ce que** la matière de doublure (7) comprend au moins une matière choisie dans un groupe comprenant une feuille, un tissu, un non-tissé et/ou un tricot.

**14.** Elément d'insertion absorbant (6) selon l'une quelconque des revendications 11 à 13, **caractérisé en ce que** l'élément d'insertion absorbant (6) est relié à d'autres éléments d'insertion absorbants (6) au moyen de la doublure (7).

Fig. 1

Fig. 2

Fig. 3

Fig. 4

Fig. 5

Fig. 6

**EP 3 145 464 B1**

**IN DER BESCHREIBUNG AUFGEFÜHRTE DOKUMENTE**

**In der Beschreibung aufgeführte Patentdokumente**

- DE 102004043289 A1
- CN 201980578 U
- US 20080167634 A1